# EUROPÄISCHE PATENTSCHRIFT

(11) **EP 0 650 354 B1**
(45) Veröffentlichungstag und Bekanntmachung des Hinweises auf die Patenterteilung: **15.12.1999**
(21) Anmeldenummer: 93914598.3
(22) Anmeldetag: 05.07.1993
(51) Int. Cl.: A61K 9/16, A61K 9/14, A61K 47/42

(54) **FESTE UND FLÜSSIGE LÖSUNGEN VON SCHWER WASSERLÖSLICHEN ARZNEISTOFFEN**
SOLID AND LIQUID SOLUTIONS OF DRUGS WHICH ARE NOT READILY SOLUBLE IN WATER
SOLUTIONS SOLIDES ET LIQUIDES DE SUBSTANCES MEDICAMENTEUSES PEU SOLUBLES DANS L'EAU

(30) Priorität: 03.07.1992 DE 4221880
(43) Veröffentlichungstag der Anmeldung: 03.05.1995
(73) Patentinhaber: ALFATEC-PHARMA GmbH, 69120 Heidelberg (DE)
(72) Erfinder: WUNDERLICH, Jens-Christian, D-69126 Heidelberg (DE); SCHICK, Ursula, D-69198 Schriescheim (DE); WERRY, Jürgen, D-67071 Ludwigshafen (DE); FREIDENREICH, Jürgen, D-69198 Schriesheim (DE)
(74) Vertreter: Winter, Brandl, Fürniss, Hübner, Röss, Kaiser, Polte, Kindermann Partnerschaft
(86) Internationale Anmeldenummer: DE9300592
(87) Internationale Veröffentlichungsnummer: WO9401090

(56) Entgegenhaltungen:
- EP-A- 0 140 255
- EP-A- 0 172 710
- EP-A- 0 282 020
- EP-A- 0 285 682
- EP-A- 0 362 582
- WO-A-91/06286
- AU-A- 495 261
- FR-A- 1 259 081
- FR-A- 2 366 835
- US-A- 3 435 110
- US-A- 3 959 472
- US-A- 4 948 580
- PATENT ABSTRACTS OF JAPAN vol. 51, no. 11 18. Juli 1981

## Beschreibung

Die vorliegende Erfindung betrifft eine schwer wasserlösliche Arzneistoffe enthaltende Zubereitung, die gekennzeichnet ist durch eine molekulardisperse bis kolloiddisperse Verteilung des schwer löslichen Arzneistoffs in einem unvernetzten hydrophilen Peptid mit einem Molekulargewichtüber 100 D.

Die Erfindung betrifft weiterhin ein Verfahren zur Herstellung solcher, schwer wasserlösliche Arzneistoffe enthaltenden Zubereitungen.

Ein weiterer Gegenstand der Erfindung sind pharmazeutische Zubereitungen, die solche Zubereitungen von schwer wasserlöslichen Arzneistoffen enthalten.

Das Problem der Lösungsvermittlung oder Solubilisation von schwer wasserlöslichen Stoffen ist beispielsweise in der pharmazeutischen Technologie hinreichend bekannt. Das Vorliegen im gelösten und undissoziierten Zustand ist wesentliche Voraussetzung für eine Resorption des Stoffes im Organismus. Um diesem Problem Abhilfe zu schaffen, existieren eine Reihe von Möglichkeiten:

Veränderungen an Molekül selbst sind Maßnahmen wie Salzbildung oder Einführung eines hydrophilen Molekülrestes. Solche Eingriffe führen zwar zu Verbindungen, die besser wasserlöslich, jedoch dissoziiert bzw. in ihrer ursprünglichen Molekülstruktur verändert sind.

Der Einsatz organischer Lösungsmittel, wie z.B. Ethanol oder Polyethylenglykolen, z.B. in flüssigen Zubereitungen ist nicht immer wünschenswert oder erfolgsversprechend, wenn man bedenkt, daß bei wäßriger Verdünnung im physiologischen Milieu die so in Lösung gehaltenen Stoffe ausfallen können und damit nicht resorptionsfähig sind.

Setzt man Tenside als Lösungsvermittler ein oder stellt wasserlösliche Komplexe (z.B. Cyclodextrin-Einschlußverbindungen) her, so muß man mögliche, nicht erwünschte Nebenwirkungen, wie beispielsweise die membranschädigende Zellwirkung solcher Stoffe, bedenken.

Zuletzt seien noch die hydrotropen Substanzen, wie Harnstoff oder N-Methylacetamid genannt, die jedoch, um eine echte lösung zu erhalten, in Konzentrationen von 20-30% angewendet werden müssen.

Gemäß EP-A-0 282 020 liegen die Wirkstoffpartikel in einer Teilchengröße von etwa 100 µm oder weniger, vorzugsweise 10 µm oder weniger vor.

AU-A-495 261 beschreibt eine vernetzte Matrix mit darin enthaltenen Wirkstoffen in Nanoform.

So kann man zusammenfassend feststellen, daß der bisherige Stand der Technik keine befriedigenden Lösungen anbietet und in vielen Fällen für besonders problematische Stoffe noch immer keine ausreichende Lösungsvermittlung gefunden wurde.

Diese Tatsache führt dazu, daß arzneiliche Zubereitungen von schwer wasserlöslichen Arzneistoffen hinsichtlich ihrer Herstellung und Anwendung in der Therapie als kritisch zu beurteilen sind.

Der Erfindung liegt daher die Aufgabe zugrunde, schwer wasserlösliche Arzneistoffe in eine solche Form zu überführen, daß der gelöste (molekulardisperse) Zustand des Arzneistoffs auf einfache Weise, ohne an die Anwesenheit organischer Lösungsmittel oder unerwünschte Nebenwirkungen verursachende Lösungsvermittler direkt gebunden zu sein, sowohl beim Lagern als auch bei der Anwendung der Zubereitung durch den Verbraucher bzw. Patienten gewährleistet ist.

Insbesondere ist eine Aufgabe der vorliegenden Erfindung, schwer wasserlösliche Arzneistoffe enthaltende Arzneimittel zu entwickeln, die die zum Stand der Technik genannten Nachteile vermeiden und daher die Wirksamkeit bzw. Bioverfügbarkeit solcher Stoffe verbessern, um eine effektive therapeutische Nutzung derartiger Pharmaka zu erreichen.

Diese Aufgabe wird durch eine schwer wasserlösliche Arzneistoffe enthaltende Zubereitung gelöst, die gekennzeichnet ist durch eine molekulardisperse bis kolloiddisperse Verteilung des schwer löslichen Arzneistoffs in einem unvernetzten hydrophilen Peptid mit einem Molekulargewicht über 100 D.

Weiterhin wird diese Aufgabe durch ein Verfahren zur Herstellung solcher, schwer wasserlösliche Arzneistoffe enthaltenden Zubereitungen gemäß Anspruch 21 gelöst. Ausführungsformen der erfindungsgemäßen Zubereitungen sowie des Verfahrens zu ihrer Herstellung werden in den Unteransprüchen genannt und beansprucht.

Schließlich wird diese Aufgabe durch pharmazeutische Zubereitungen gelöst, die solche Zubereitungen von schwer wasserlöslichen Arzneistoffen enthalten.

Die vorliegende Erfindung beruht nun auf der völlig überraschenden Feststellung, daß allein ein hydrophiles Peptid mit einem Molekulargewicht über 100 D ausreichend ist, schwer wasserlösliche Arzneistoffe in gelöster Form so zu stabilisieren, daß keine Zusätze von organischen Lösungsmitteln bzw. Lösungsvermittlern mehr notwendig sind.

Derartige erfindungsgemäße Systeme können als flüssige, wäßrige Lösungen vorliegen, aber es kann auch durch geeignete Verfahren das Lösungsmittel entzogen werden (z.B. Sprüh- oder Gefriertrocknung). Die so getrockneten Produkte lassen sich durch Wasserzusatz bzw. im physiologischen Milieu wieder zu ihrem Ausgangszustand auflösen (redispergieren).

Das Ausmaß und die arzneistoffspezifischen Grenzkonzentrationen, bei denen der molekulardisperse Anteil in ein disperses System übergeht, läßt sich durch Auswahl geeigneter hydrophiler Peptide mit einen besonders hohen Anteil lipophiler Bereiche etc., aber auch durch Zusätze, die die Helakilität des hydrophilen Peptids beeinflussen können, wie z.B. Polythylenglykole, Tenside etc., einstellen.

In diesem Sinne kann daher der Arzneistoff in der erfindungsgemäßen Form auch gleichzeitig teils molekulardispers teils in kolloiddisperser Form (Nanosole, die in zahlreichen Patentanmeldungen der ALFATEC-Pharma GmbH ausführlich beschrieben werden) vorliegen.

Durch Zusätze von Weichmachern, ausgewählt aus der Gruppe bestehend aus: Glycerol, Propylenglykol, Polyethylenglykole, Triacetin, Sorbitol, Sorbitangemische, Sorbitollösungen, Glucosesirup, Polyole, Zuckeralkohole; sowie deren Mischungen lassen sich ferner halbfeste bis gelförmige Konsistenzen einstellen, wie sie von klassischen Weichgelatinekapseln oder den in zahlreichen Patentanmeldungen der ALFATEC-Pharma GmbH beschriebenen Cryopellets bekannt sind.

Unter einem hydrophilen Peptid im Sinne der Erfindung versteht man eine aus Aminosäuren oder Derivaten von Aminosäuren aufgebaute Substanz natürlichen, synthetischen oder partialsynthetischen Ursprungs mit einem Molekulargewicht über 100 D.

Insbesondere kann es sich um Kollagen, Kollagenderivate oder von Kollagen abgeleitete Substanzen aus der folgenden Gruppe handeln: Gelatine, fraktionierter Gelatine, Kollagenhydrolysate, Gelatinederivate; sowie deren Mischungen.

Erfindungsgemäß sind auch hydrophile Peptide, wie beispielsweise Pflanzenproteine, Pflanzenproteinhydrolysate, Elastinhydrolysate, Albumine, Caseinhydrolysate, Casein; sowie deren Mischungen in diese Definition einbezogen.

Es hat sich weiterhin gezeigt, daß die Verwendung eines hydrophilen Peptids als lösungsvermittelnde Substanz für schwer wasserlösliche Arzneistoffe nicht nur den Vorteil einer Stabilisierung des Arzneistoffs in wäßriger Lösung bietet, sondern darüberhinaus noch weiterreichenden Nutzen aufweist.

Der Arzneistoff liegt in einer erfindungsgemäßen Zubereitung einerseits geschützt vor äußeren Einflüssen wie z.B. Licht, Luftsauerstoff etc. vor. Liegt die erfindungsgemäge Zubereitung in fester, wiederauflösbarer Form vor, ist darüberhinaus ein Schutz empfindlicher Arzneistoffe vor Feuchtigkeit bzw. Feuchtigkeitszutritt gewährleistet. Damit sind solche Arzneistoffe in ihren Zubereitungen gleichzeitig wirksam vor Aktivitätsverlusten durch Zersetzungsprozesse bewahrt.

Weiterhin können hydrophile Peptide, insbesondere von Kollagen abgeleitete Substanzen vorteilhafterweise einen unangenehmen Geschmack des schwer wasserlösliche Arzneistoffes in einer erfindungsgemäßen Zubereitung überdecken. Diese Geschmacksüberdeckung kann noch verstärkt werden durch Zusatz von Sorbitol, der neben seiner Eigenschaft als Weichmacher vorteilhaft die Funktion eines Süßstoffs mit nicht-kariogener Eigenschaft besitzt.

Um die physiologischen Hintergründe der Resorption von Arzneistoffen im allgemeinen und die verbesserte Resorptionsquote der erfindungsgemäßen Pelletformulierungen ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:
a) die Gastrointestinalmembran wirkt als Lipidbarriere,
b) der Arzneistoff wird nur in gelöster und ungeladener, d.h. nichtionisierter Form aufgenommen,
c) saure Arzneistoffe werden bevorzugt im Magen, basisische Arzneistoffe bevorzugt im Darm resorbiert.

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Fig. 3), nämlich
- durch die Mucus-Schicht und eine wässerige, daran anhärierende Schicht
- die Zellmembranen der intestinalen Epithelzellen mit der daran kovalent gebundenen Glykocalix sowie
- die sogenannten "Tight Junctions", die die Epihelzellen an ihrer apikalen Seite miteinander verbinden.

Diese Barrieren bedingen, daß die Resorption von Arzneistoffen hauptsächlich abhängig von ihrem Verteilungsmechanismus und Ladungszustand - durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Muscus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden. Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminsäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phosholipid-Doppelschichten mit ihrem lipophilen Anteil stellen eine weitere Barriere für den Transport der zu resorbierenden Arzneistoffe dar.

Offensichtlich liegt der Arzneistoff in einer erfindungsgemäßen Formulierung sowohl in molekulardisperser (gelöster) Form, aber auch in einer Konjugatbindung mit dem hydrophilen Peptid vor, so daß die bekannten Resorptionsbarrieren besser überwunden werden und Resorption bzw. Bioverfügbarkeit einer erfindungsgemäßen Zubereitung deutlich gesteigert werden.

Eigene Untersuchungen haben gezeigt, daß eine solche Steigerung gegenüber herkömmlichen Zubereitungen bei der Applikation in vivo zu erreichen ist.

Somit läßt sich zusammenfassend sagen, daß die vorliegende Erfindung eine pharmazeutische Zubereitung schwer wasserlöslicher Arzneistoffe zur Verfügung stellt, die dadurch gekennzeichnet ist, daß das Ausmaß der in vivo Resorption des schwer wasserlöslichen Arzneistoffes aus einer molekulardispersen Verteilung in einem hydrophilen Peptid mit einem Molekulargewicht über 100 D, im Vergleich zu herkömmlichen Zubereitungen um 50% bis 100% gesteigert wird.

Neben einer gesteigerten Resorption ist ebenfalls eine erhöhte Blutplasmakonzentration sowie ein schnellerer Anstieg derselben, im Vergleich zu handelsüblichen Präparaten zu beobachten.

Die gesteigerte Resorptionsrate des Arzneistoffes aus einer erfindungsgemäßen Zubereitung läßt sich weiterhin sehr eindrucksvoll zeigen, wenn man beispielsweise den peroralen Applikationsweg einer pharmazeutischen Zubereitung betrachtet:

Nach Wiederauflösen einer erfindungsgemäßen Zubereitung im physiologischen Milieu bleibt die molekulardisperse Verteilung eines schwer wasserlöslichen Arzneistoffes erstaunlicherweise erhalten. Die Freigabe des Arzneistoffs aus seiner Zubereitung erfolgt ohne vorgelagerten Gleichgewichtsprozeß, im Gegensatz zu herkömmlichen, Solubilisatoren enthaltenden Zubereitungen. Weiterhin sind Ausfällungen bzw. Ausflockungen durch die mit den zu resorbierenden Substanzen gebildeten inter- und intramolekularen Konjugate bzw. Einlagerungen aus Gelatine bzw. einer von Kollagen abgeleiteten Substanz wirksam verhindert. Diese umhüllende (viskose) Solschicht schützt den Arzneistoff beispielsweise vor physiologischen Einflüssen, sodaß er nicht aus dem Konjugat mit der Gelatine verdrängt wird. Auf diese Weise wird vorteilhaft der Erhalt der molekulardispersen Verteilung des Arzneistoffs von der Freisetzung aus der Arzneiform bis zur nachfolgenden Resorption sichergestellt. Dieser Schutz kann noch verstärkt werden, indem in die Zubereitung pharmazeutisch übliche Hilfsstoffe, wie beispielsweise Puffersubstanzen (z.B. Dinatrium-hydrogenphosphat) eingearbeitet werden.

Desweiteren zeigt eine im physiologischen Milieu schmelzende erfindungsgemäße Masse aus einem schwer wasserlöslichen Arzneistoff und Gelatine eine hohe Affinität zu Schleimhautoberflächen. Dieses Anheften oder Ankleben an der Schleimhaut (Bioadhäsion) bewirkt einen direkten Kontakt des Arzneistoffes mit der physiologischen Resorptionsbarriere.

Überraschenderweise werden Arzneistoffe, die in herkömmlicher, kristalliner Form aufgrund ihrer schlechten Benetzbarkeit Bioverfügbarkeitsprobleme aufweisen, aus einer festen bzw. trockenen, erfindungsgemäßen Zubereitung ohne solche Probleme im physiologischen Milieu wiederaufgelöst und stehen in molekulardisperser Form für die Resorption zur Verfügung. Aus diesem Grund kann auf den Einsatz größerer Mengen von Tensiden oder Netzmitteln, wie es für herkömmliche Zubereitungen notwendig und üblich ist, um schwer wasserlösliche Arzneistoffe überhaupt ausreichend benetzbar zu machen, verzichtet werden.

Erstaunlicherweise finden aufgrund der molekulardispersen Verteilung des Arzneistoffs in den erfindungsgemäßen Zubereitungen keinerlei Änderungen von Kristalimodifikationen eines Arzneistoffs mehr statt. So kann die Erfindung vorteilhafterweise auch für solche Arzneistoffe eingesetzt werden, die während der Herstellung bzw. Lagerung einer entsprechenden Arzneiform zu Modifikationsänderungen neigen und deshalb Bioverfügbarkeitsprobleme zur Folge haben.

Der Lösevorgang aus einer erfindungsgemäßen Zubereitung als zeitbestimmender Faktor hängt ausschließlich von der Art und Zusammensetzung des ausgewählten hydrophilen Peptids ab und ist in der Freisetzung modulierbar. So können Akutformen, wie auch Retardformen formuliert werden.

Gelatine ist ein aus kollagenhaltigem Material gewonnenes Skleroprotein, das je nach Herstellungsprozeß unterschiedliche Eigenschaften hat. Sie besteht im wesentlichen aus vier Molekulargewichtsfraktionen, die die physikalisch-chemischen Eigenschaften in Abhängigkeit vom Molekulargewicht und prozentualem Gewichtsanteil beeinflussen. Je höher z.B. der Anteil Mikrogel (10⁷ bis 10⁸ D) liegt, desto höher ist auch die Viskosität der wäßrigen Lösung. Handelsübliche Sorten enthalten bis zu 15 Gewichtsprozent Mikrogel. Die Fraktionen der alpha-Gelatine und deren Oligomere (9,5 x 10⁴/10⁵ bis 10⁶ D) sind entscheidend für die Gelfestigkeit und liegen üblicherweise zwischen 10 und 40 Gewichtsprozent. Molekulargewichte unterhalb der alpha-Gelatine werden als Peptide bezeichnet und können in herkömmlichen Gelatinequalitäten (niedrigbloomig) bis zu 80 Gewichtsprozent betragen.

Gelatine besitzt ein temperatur- und konzentrationsabhängiges Sol-Gel-Umwandlungsverhalten, das von der molekularen Zusammensetzung abhängig ist. Als Konventionsmethode für das Gelbildungsvermögen wird die Bloomzahl angegeben. Niedrige Handelsqualitäten beginnen bei 50 Bloom, hochbloomige Sorten liegen bei etwa 300 Bloom.

Die chemischen und physikalischen Eigenschaften variieren je nach Herstellungsverfahren, wobei besonders schonend gewonnene Gelatinesorten (geringer Anteil an rechtsdrehenden Aminosäuren und Peptiden) kurze Sol-Gel-Umwandlungsgeschwindigkeifen und Schmelzpunkte oberhalb 37°C (gemessen als 10%ige Lösung) aufweisen. Bei besonders schonender Herstellungsweise bleibt die Sekundär- und Tertiärstruktur des Kollagens in der Gelatine weitgehend erhalten.

Fraktionierte Gelatine stellt den Spezialfall von Gelatine dar und wird durch spezielle Herstellungstechniken, wie z.B. Ultrafiltration aus herkömmlicher Gelatine gewonnen. Die Zusammensetzung kann z.B. durch Entfernung von Peptiden (MG < 9,5 x 10⁴ D) oder durch Mischungen aus Einzelfraktionen wie z.B. alpha-Ketten, dimerea und trimeren Ketten oder Mikrogel varriiert werden.

Darüberhinaus hat Gelatine bzw. fraktionierte Gelatine gute Tensideigenschaften mit Schutzkolloidwirkung und Emulgatoreigenschaft.

Kollagen in nativer Form ist wasserunlöslich. Durch spezielle Herstellungsverfahren gibt es heute lösliche Kollagentypen mit einem durchschnittlichen Molekulargewicht von ca. 300.000 D.

Gelatinederivate sind chemisch veränderte Gelatinen, wie z.B. succinylierte Gelatine oder Oxypolygelatine, die auch als Plasmaexpander bekannt sind. Solche Plasmaexpander können auch spezielle Elektrolytzusätze enthalten.

Unter Kollagenhydrolysat wird ein von Kollagen oder Gelatine druckhydrolytisch oder enzymatisch gewonnenes Produkt verstanden, das kein Sol-Gel-Umwandlungsvermögen mehr aufweist.

Kollagenhydrolysate sind leicht kaltwasserlöslich und die Molekulargewichtszusammensetzung kann zwischen einigen Hundert D bis unterhalb von 9,5 x 10⁴ D liegen. Auf enzymatischem Wege gewonnene Produkte sind in der molekularen Zusammensetzung homogener und zeigen noch gute Tensid- und Emulgatorwirkung.

Neuentwickelte Produkte stellen die Pflanzenproteine und deren Hydrolysate dar, die in ihren Eigenschaften weitgehend den Kollagenhydrolysaten entsprechen. Sie werden vorzugsweise aus Weizen und Soja gewonnen und besitzen beispielsweise Molekulargewichte von 200.000-300.000 D bzw. 1.000-10.000 D.

Elastinhydrolysate werden enzymatisch aus Elastin gewonnen und bestehen aus einer einzigen Polypeptidkette mit einem hohen Anteil an nichtpolaren Aminosäuren. Sie können daher auch in lipophilen Systemen verwendet werden. Elastinhydrolysate weisen ein Molekulargewicht von 2.000 - 3.000 D auf und sind auf der Haut stark filmbildend.

Der Arzneistoff kann in den helikalen Bezirken des hydrophilen Peptids z.B. durch lipophile Wechselwirkungen, Wasserstoffbrücken, ionische Wechselwirkungen, polare Wechselwirkungen, Dipol-Dipol-Wechselwirkungen etc. molekulardispers stabilisiert werden.

Die Helikalität ist von der molekularen Zusammensetzung, der Polymolekularität, dem Racemisierungsgrad der alpha-C-Atome in den Peptideinheiten, dem Gehalt an Prolin bzw. Hydroxyprolin und dem Gehalt an helikophoben Aminosäuren abhängig. Diese Parameter können durch geeignete Herstellungsverfahren in weiten Grenzen beeinflußt werden.

Bei Wiederauflösen einer erfindungsgemäßen Zubereitung rekonstituieren sich diese helikalen Strukturen sehr schnell, vor allem im physiologischen Milieu bei 37°C. Dies geschieht bereits, wenn die vorliegenden Konzentrationen des hydrophilen Peptids sehr gering sind.

Durch ein derartiges Ordnungsprinzip im molekularen Bereich bleibt der Arzneistoff in molekulardisperser Form an der Helix angelagert (Konjugatbildung) und wird so wirksam vor Ausfällungen bzw. Ausflockungen geschützt. Daher kann ein Arzneistoff aus einem solchen, stabilisierten, erfindungsgemäßen System idealerweise direkt resorbiert werden.

Bei Arzneistoffen, die extrem thermolabil sind, ermöglicht die Erfindung in einer weiteren Ausführungsform vorteilhafterweise, Zubereitungen mit erfindungsgemäßen Eigenschaften zur Verfügung zu stellen, die unter ausschließlich kalten Bedingungen, d.h. bei Raumtemperatur ohne Anwendung von Wärme hergestellt sind, z.B. durch Lyophilisation. Bei dieser Vorgehensweise kann man ein hydrophiles Peptid, ausgewählt aus der Gruppe bestehend aus: Pflanzenproteine, Pflanzenproteinhydrolysate, Elastinhydrolysate, Kollagenhydrolysate, kaltwasserlösliche Gelatine, Gelatinederivate; sowie deren Mischungen; mit einem Maximum der Molekulargewichtsverteilung unterhalb 10⁵ D, verwenden.

Darüberhinaus stellt diese Ausführungsform der Erfindung eine Zubereitung zur Verfügung, die sich besonders schnell in kaltem Wasser auflöst, in der Regel innerhalb von 2 min. Eine solche Zubereitung eignet sich für pharmazeutische Akutformen und Parenteralia, sowie zur Herstellung von Trinklösungen oder Instantzubereitungen zur innerlichen und äußerlichen Anwendung.

Erfindungsgemäß können auch hydrophile Peptide verwendet werden, die sol-gel-bildende Eigenschaften aufweisen, wie beispielsweise Gelatine oder fraktionierte Gelatine. Solche Substanzen können in Abhängigkeit ihrer molekularen Zusammensetzung beispielsweise für feste Zubereitungen eingesetzt werden, die den Arzneistoff schnell oder langsam in wäßrigem Medium bei 37°C freisetzen.

Langsam freisetzende Formulierungen können auch zur Herstellung einer den Arzneistoff linear freisetzenden Retardform verwendet werden.

Für solche Retardformulierungen können neben den hydrophilen Peptiden, übliche Hilfsstoffe zur Retardierung verwendet werden, z.B. Alginate, Cellulosederivate, Polyvinylpyrrolidon, natürliche oder modifizierte Stärken, Polyacrylsäure und Polymere aus Methacrylsäure und Methacrylsäureestern, hydrophile Gummen etc.; sowie deren Mischungen.

Neben der Verwendung zu Retardierungszwecken können diese Stoffe, bei Zusatz in geringer Menge, insbesondere zu Gelatine, noch weitere Effekte bewirken. Sie besitzen beispielsweise die Eigenschaft, die Tripelhelices von Gelatine miteinander zu verknüpfen. Geschieht dies nur an wenigen Punkten, bleiben die Eigenschaften des Makromoleküls Gelatine in einer derartigen Form erhalten, daß der Arzneistoff in den Molekülverbänden in molekulardisperser Form wirksam stabilisiert und fixiert wird.

Weiterhin lassen sich helikale Strukturen des hydrophilen Peptids, z.B. von Gelatine oder fraktionierter Gelatine, durch Zusätze wie z.B. Weichmacher, Tenside etc. stark beeinflussen. So fördert z.B. Glycerin, Polyethylenglykole etc. die Ausbildung helikaler Konformationen und trägt ebenfalls vorteilhaft zur Stabilisierung eines erfindungsgemäßen molekulardispersen Systems bei.

Überraschenderweise hat sich zusätzlich gezeigt, daß eine Kombination von Gelatine mit Weichmachern als sogenannter "Enhancer" (Resorptionsbeschleuniger) wirkt. Dadurch wird der Resorptionsprozeß eines Arzneistoffes erheblich erleichtert bzw. beschleunigt und vor allem signifikant gesteigert gegenüber den Zubereitungen des Standes der Technik.

Daher stellt die vorliegende Erfindung auch einen Enhancer zur Verfügung, der dadurch gekennzeichnet ist, daß er eine Kombination wenigstens eines hydrophilen Peptids, ausgewählt aus der Gruppe bestehend aus: Kollagen, Kollagenderivate, Gelatine, fraktionierter Gelatine, Kollagenhydrolysate, Gelatinederivate, Pflanzenproteine, Pflanzenproteinhydrolysate, Elastinhydrolysate, Albumine, Caseinhydrolysate; Caseine; sowie deren Mischungen; mit einem Weichmacher, der ausgewählt ist aus der Gruppe bestehend aus: Glycerol, Propylenglykol, Polyethylenglykole, Triacetin, Sorbitol, Sorbitangemische, Sorbitollösungen, Glucosesirup, Polyole, Zuckeralkohole; sowie deren Mischungen, enthält.

Eine weitere Ausführungsform der vorliegenden Erfindung ergibt sich insbesondere bei schwer wasserlöslichen Peptidarzneistoffen, wie z.B. Renin-Antagonisten. Da Peptidarzneistoffe nach der Applikation im Magen-Darm-Trakt einem erhöhten enzymatischen Abbau (Inaktivierung noch vor der Resorption) durch physiologische Enzyme unterliegen, versucht man durch Derivatisierung, z.B. durch Einführung von Schutzgruppen, den Peptidarzneistoff gegenüber den gastrointestinalen Enzymen zu stabilisieren. Dadurch steigt aber der lipophile Charakter solcher Arzneistoffmoleküle an, verbunden mit einer Verschlechterung ihrer Wasserlöslichkeit.

Solche derivatisierten Peptidarzneistoffe können zur Herstellung einer erfindungsgemäßen, molekulardispersen Verteilung besonders geeignet sein. Wechselwirkungen lipophiler Molekülteile mit lipophilen Bezirken in den helikalen Strukturen des hydrophilen Peptids lassen sich dabei vorteilhaft zur Stabilisierung des molekulardispersen Systems ausnutzen.

Solche erfindungsgemäßen Zubereitungen, die Peptidarzneistoffe enthalten, können vorteilhaft gleichzeitig übliche Penetrationsbeschleuniger (Enhancer) oder Proteaseinhibitoren enthalten.

Herkömmliche Penetrationsbeschleuniger können beispielsweise in folgende Gruppen unterteilt werden:
- Chelatoren, wie z.B. Ethylendiamintetraessigsäure (EDTA), Citronensäure, Salicylate, N-Acylderivate von Kollagen oder von Kollagen abgeleiteten Substanzen, N-Amino-acylderivate von beta-Diketonen (Enamine);
- Surfactants, wie z.B: Natriumlaurylsulfat, Polyoxyethylen-9-laurylether, Polyoxyethylen-20-cetylether;
- Non-Surfactants, wie z.B. ungesättigte zyklische Harnstoffverbindungen, 1-Alkyl- und 1-Alkenyl-azacycloalkanon-Derivate;
- Gallensaure Salze und Derivate, wie z.B. Natrium-deoxycholat, Natrium-glykocholat, Natrium-taurodihydrofusidat (STDHF), Natrium-glykodihydrofusidat;
- Fettsäuren und Derivate, wie z.B. Ölsäure, Caprylsäure, Caprinsäure, Acylcarnitine, Acylcholine und ihre Mono- und Diglyceride;

Auf diese Art und Weise kann, besonders im Falle von Peptidarzneistoffen, ihre bekannt niedrige Resorptionsquote im Gastrointestinaltrakt noch weiter erhöht werden, als es mit einer erfindungsgemäßen Zubereitung ohne zusätzliche Penetrationsbeschleuniger ohnehin schon möglich ist.

Das hydrophile Peptid, wie z.B. Gelatine besitzt dabei noch eine weitere Eigenschaft. Schleimhautirritierende Wirkungen von üblichen Penetrationsbeschleunigern können wirksam vermindert werden.

Enthalten erfindungsgemäße Zubereitungen Weichmacher, können diese eine halbfeste bis gelförmige Konsistenz aufweisen und können sowohl in Form von Cryopellets, z.B. als einzeldosierte Arzneiform, als auch für Kapselfüllungen, z.B. in Weichgelatinekapseln verwendet werden.

Konventionelle Kapselfüllrezepturen bestehen in vielen Fällen aus Lösungen von schwer löslichen Arzneistoffen in organischen Lösungsmitteln, kompliziert zusammengesetzten Lösungsmittelgemischen und Lösungsvermittlern wie z.B. Polyethylenglykole, 1,2-Propylenglykol, 1,3-Butandiol, 1,4-Butandiol, 1,2-Propandiol, ein Polyoxyethylenpolyoxypropylencopolymerisat, Tetrahydrofurfurylalkohol und andere mehrwertige Alkohole.

Setzt man nun erfindungsgemäße Systeme als Füllungen für herkömmliche Weichgelatinekapseln ein, so ergeben sich folgende Vorteile:
- Herkömmliche, kompliziert zusammengesetzte Lösungsmittelgemische (siehe oben) können mengenmäßig reduziert werden bzw. auf einzelne Komponenten kann ganz verzichtet werden.
- Es erfolgen keine Ausflockungen bzw. Ausfällungen von schwer wasserlöslichen Arzneistoffen, wie sie bei herkömmlichen Füllrezepturen im physiologischen Milieu auftreten können.

Bekannte, pharmazeutische Stoffe, die als Lösungsvermittler verwendet werden, können durch die vorliegende Erfindung in ihrer Menge reduziert werden bzw. in Kombination mit dem erfindungsgemäßen hydrophilen Peptid zu neuen Eigenschaften der pharmazeutischen Formulierung führen. So können selbst insbesondere Stoffe, wie z. B. Polyethylenglykol 200 oder 400, Tetrahydrofurfurylalkoholpolyethylenglykolether oder 1,2-Propylenglykol zu diesen neuen Eigenschaften führen.

Ebenso können auch Arzneistoffe, die nicht oder nur in geringem Anteil in Lösung gebracht werden können, in eine Form mit neuen Eigenschaften überführt werden, wobei der Arzneistoff z. T. in erfindungsgemäß gelöster (molekulardisperser) Form als auch gleichzeitig in kolloiddisperser Form vorliegen kann. Das gleiche gilt auch für Emulsionen.

Tenside gehen mit hydrophilen Peptiden, wie z.B. Gelatine oder Kollagenhydrolysaten starke Wechselwirkungen ein, z.B. Komplexe. Diese Komulexbildung ist umso stärker, je polarer beispielsweise ein Tensidanion (z.B. Natriumlaurylsulfat, Natriumdroctylsulfosuccinat) ist. Daraus ergibt sich, daß die erfindungsgemäße, solubilisierende Eigenschaft der hydrophilen Peptide mit der von Tensiden sinnvollerweise kombiniert werden kann.

Im gleichen Sinne wie Tenside können hydrotrope Stoffe wie z.B. Harnstoff, N-Methylacetamid oder Nicotinsäure erfindungsgemäß verwendet werden.

Die Anwesenheit von Gelatine bzw. einer von Kollagen abgeleiteten Substanz als Makromolekül primär biogenen Ursprungs stellt darüberhinaus die größtmögliche Verträglichkeit einer erfindungsgemäßen Arzneizubereitung im Organismus sicher. Unerwünschte Nebenwirkungen von Hilfsstoffen, z.B. Irritationen von Schleimhäuten, wie sie etwa durch organische Lösungsmittel oder Tenside hervorgerufen werden können, fallen so ganz weg oder können zumindest entscheidend reduziert werden.

Bekanntermaßen besitzt Gelatine je nach Herstellungsverfahren einen isoelektrischen Punkt im sauren (Gelatine Typ B) oder im alkalischen Bereich (Gelatine Typ A). Diese Eigenschaft kann erfindungsgemäß zur direkten Bildung von Mikro- bzw. Nanokapseln in einer molekulardispersen Verteilung von Arzneistoff und beispielsweise von Kollagen abgeleiteten Substanzen ausgenutzt werden. So können bei Verwendung von Gelatinen mit entgegengesetzter Ladung in Mischung mit arzneistoffhaltiger Lösung (z.B. bei einem pH von 6-7) durch Entfernung des Lösungsmittels Mikrokapseln hergestellt werden.

Gelatinen oder Kollagenhydrolysate können weiterhin mit einem Arzneistoff z.B. unter einem ca. 2-3%igen Zusatz von Salzen Konjugate bilden.

Damit stellt die vorliegende Erfindung einen überaus wertvollen Beitrag zur sinnvollen therapeutischen Nutzung und Anwendung erfindungsgemäßer schwer wasserlöslicher Arzneistoffe enthaltenden Pharmaka dar.

Die Begriffe "Wirkstoffe" und "Arzneistoffe" werden im Rahmen der vorliegenden Erfindung synonym gebraucht und umfassen definitionsgemäß sowohl Substanzen, die biologisch aktiv sein können und nicht oder noch nicht therapeutisch eingesetzt werden, wie auch Arzneistoffe, die dem Organismus zugeführt werden und mit diesem in Wechselwirkung treten.

Unter schwer wasserlöslichen Arzneistoffen im Sinne der vorliegende Erfindung versteht man Arzneistoffe, die Stoffeigenschaften wie geringe Löslichkeit, schlechte Benetzbarkeit, metastabile Modifikationen aufweisen und dadurch zu Bioverfügbarkeitsproblemen führen.

Unter dem Begriff "schwer wasserlöslich" werden im Sinne der vorliegenden Erfindung die in den Pharmakopoen beschriebenen Begriffe für Löslichkeiten wie "wenig löslich", "schwer löslich", "sehr schwer löslich" bis "praktisch unlöslich" zusammengefaßt.

Der Begriff "Arzneistoff" wird erfindungsgemäß wie folgt definiert:

Arzneistoffe können synthetischen, partialsynthetischen oder natürlichen Ursprungs sein, sowohl chemisch einheitliche Substanzen oder Substanzgemische sein, als auch Kombinationen aus verschiedenen pharmakologisch wirksamen Komponenten. Ferner soll der Arzneistoffbegriff aber auch Phytopharmaka und Pflanzenextrakte allgemein umfassen und schließlich auch Hormone, Vitamine und Enzyme miteinbeziehen.

Auch enantiomerreine Wirkstoffe oder Pseudoracemate sind erfindungsgemäß geeignet. Weiterhin können Wirkstoffe aus dem Bereich der diätetischen Lebensmittel (health care) sowie aus dem Bereich der Kosmetik verwendet werden.

Im Falle für die Erfindung geeigneter, schwer wasserlöslicher Arzneistoffe besteht keinerlei Begrenzung bzgl. der chemischen Substanzklassen. Im folgenden werden beispielhaft chemische Substanzklassen und einige zugehörige Vertreter genannt:
1. Phenyl-ethylamin-Derivate, wie z.B., Salbutamol, Chloramphenicol;
2. Phenyl-propylamin-derivate, wie z.B. Haloperidol;
3. Phenyl-butylamin-Derivate, wie z.B. Fluspirilen, Verapamil;
4. Arylalkansäure-Derivate, wie z.B. Diclofenac, Indometacin;
5. Diphenylmethan-Derivate, wie z.B. Chlorphenoxamin, Diphenhydramin;
6. Dibenzocycloheptadiene und Dibenzocycloheptatriene, wie z.B. Amitriptylin;
7. Steroid-Derivate, wie z.B. Fuocortolon, Cortisonacetat;
8. Phenolether, wie z.B. Bezafibrat, Etacrynsäure;
9. 4-Amninobenzoesäure-Derivate, wie z.B. Bromhexin;
10. Anilid-Derivate, wie z.B. Paracetamol;
11. Anilin-Derivate, wie z.B. Mefenaminsäure;
12. Aromatische Carbonsäuren und Derivate, wie z. B. Acetylsalicylsäure;
13. Aryloxypropylamin-Derivate, wie z.B. Propranolol;
14. Sulfonamid-Derivate, wie z.B. Sulfaguanidin, Furosemid, Sulfamethoxazol;
15. Sulfonylharnstoff-Derivate, wie z.B. Glibenclamid;
16. Beta-Lactam-Antibiotika mit Benzylpenicillin-, Phenoxymethylpenicillin- bzw. Cefalosporin C-Grundstruktur, wie z.B. Penicillin V, Amoxicillin;
17. Furan-Derivate, wie z.B. Nitrofurantoin;
18. Tetrahydrofuran-Derivate, wie z.B. Mefrusid;
19. Pyrazol-Derivate, wie Pyrazolinon-Derivate oder Pyrazolidin-3,5-dione, wie z.B. Oxyphenbutazon;
20. Imidazol-Derivate, wie z.B. Clotrimazol, Cimetidin, Benzimidazole wie z. B. Omeprazol;
21. Imidazolidin-Derivate, wie z.B. Phenytoin;
22. 1,3,4-Thiadiazol-Derivate, wie z.B. Acetazolamid;
23. Pyridine, wie z.B. Nifluminsäure;
24. Piperidin-Derivate, wie z.B. Pethidin;
25. Isochinolin-Derivate, wie z.B. Papaverin;
26. Thioxanthen-Derivate, wie z.B. Chlorprothixen;
27. Pyrimidin-Derivate, wie z. B. Brivudin, Hexahydropyrimidine, Uracile, Barbitursäurederivate, wie z.B Allopurinol, Secbutabarbital, weiterhin Pyrazin-, Piperazin-, Chinazolin-Derivate;
28. Phenothiazin-Derivate und Azaphenothiazinderivate, wie z.B. Chlorpromazin;
29. 1,2,4-Benzothiadiazine, wie z.B. Hydrochlorothiazid;
30. Dibenzoazepin-Derivate, wie z.B. Imipramin;
31. Benzodiazepin-Derivate, wie z.B. Diazepam, Oxazepam; Chlordiazepoxid;
32. Purine, wie z.B. Theophyllin;
33. Pteridine, wie z.B. Methotrexat, Triamteren;
34. Salpetersäureester-Derivate, wie z.B. Isosorbiddinitrat;
35. Peptidarzneistoffe, wie z.B. Renin-Antagonisten;

Weiterhin Arzneistoffe aus den Gruppen Derivate von aliphatischen Carbonsäuren, Naphthalin-Derivate, Anthracen-Derivate, Pyrrol-Derivate, Pyrrolidin-Derivate, sowohl Pyrrolidinon- als auch Pyrrolidindion-Derivate, Thiophen-Derivate, Isoxazol-Derivate, Oxazol- und Oxazolidin-Derivate, 2-Imidazolin-Derivate, Benzimidazol-Derivate, 1,3-Thiazol-, 1,3-Thiazolidin-, 1,2,5-Thiadiazol-Derivate, Chroman-Derivate, Chinolin-Derivate, Morpholine, Morphinähnliche, Aminoalkohole, Guanidin-und Biguanidderivate, Oxazaphosphorine, Adamantan-Derivate, aus Isopren-Einheiten aufgebaute Stoffe natürlichen, partialsynthetischen oder synthetischen Ursprungs, etc.

Es können aber auch allgemein organische oder anorganische wie auch metallorganische Substanzen sein, bei welchen eine Lösungsvermittlung sinnvoll erscheint.

So können die erfindungsgemäßen Formulierungen auch vorteilhaft z. B. bei der Herstellung von photographischen Schichten eingesetzt werden.

Im folgenden wird das Verfahren zur Herstellung einer erfindungsgemäßen, schwer wasserlösliche Arzneistoffe enthaltenden Zubereitung beschrieben.

Weitere Ausführungen hierzu sind in den "Feste und flüssige Lösungen von Flavonoiden" (11AL2725), "Pharmazeutische Zubereitung mit der Wirkung des Gingko biloba-Extrakts" (11AL2728) und "Feste und flüssige Lösungen von Gingko biloba-Extrakt" (11AL2726) betitelten Patentanmeldungen der ALFATEC Pharma GmbH, gegebenenfalls der GGU mbH & Co. KG vom selben Tage, deren Inhalte auch zum Gegenstand der Offenbarung der vorliegenden Patentanmeldung gemacht werden, beschrieben.

Im einfachsten Fall läßt sich das erfindungsgemäße Verfahren zur Herstellung einer schwer wasserlösliche Arzneistoffe enthaltenden Zubereitung mit den folgenden Verfahrensschritten beschreiben:
a) Man dispergiert den Arzneistoff in einem Lösungsmittel in molekularer oder kolloidaler Form und vermischt diese Dispersion mit einer wässrigen Lösung eines hydrophilen Peptids mit einem Molekulargewicht über 100 D.
b) Aus dieser Lösung werden das oder die Lösungsmittel entfernt.

Üblicherweise beträgt das Mengenverhältnis von Arzneistoff zu dem hydrophilen Peptid 1:0,5 bis 1:1000, insbesondere aber 1:2 bis 1:50, angegeben in Gewichtsteilen Trockensubstanz.

Diesem erfindungsgemäßen System können weitere zusätzliche hydrophile Makromoleküle, die die lösungsvermittelnde Wirkung des hydrophilen Peptids verstärken , u.U. sogar potenzieren können, wie z.B. Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylalkohol, Polyvinylpyrrolidon, Polyethylenglykole, Polyacrylsäure, und Polymere aus Methacrylsäure und Methacrylsäureestern; sowie deren Mischungen, zugesetzt werden.

Das Gewichtsverhältnis dieser hydrophilen Makromoleküle zu dem hydrophilen Peptid kann bis zu 1:1 betragen.

Dem nach dem beschriebenen erfindungsgemäßen Verfahren hergestellten System können ggf. weitere für die entsprechende Verwendung geeignete Träger- und Hilfsstoffe zugesetzt werden. Solche können z.B. pharmazeutisch übliche Träger- und Hilfsstoffe aus folgenden Gruppen sein:
1. zusätzliche Gerüstbildner,
   z.B. Dextrane, Saccharose, Glycin, Lactose, Sorbitol, Polyvinylpyrrolidon, Mannit; sowie Mischungen davon.
2. Füllstoffe, z.B. Stärke.
3. Tenside, z.B. Polysorbate
4. pH-Korrigentien bzw. Puffersubstanzen, z.B. Dinatriumcitrat, Dinatriumphosphat etc.
5. Farbstoffe, z.B. Curcumin
6. aromatisierende Zusätze bzw. Geschmackskorrigentien, z.B. Fruchtauszüge, Fruchtsaftkonzentrate etc.

In einer Ausführungsform des unter a) beschriebenen Verfahrensschrittes kann der schwer wasserlösliche Arzneistoff in mit Wasser mischbaren, organischen Lösungsmitteln gelöst oder suspendiert werden. Wasser kann bereits zugegen sein, ist aber nicht zwingend notwendig.

Als organische Lösungsmittel für den schwer wasserlöslichen Arzneistoff können beispielsweise mit Wasser mischbare organische Lösungsmittel ausgewählt werden, aus der Gruppe bestehend aus: mit Wasser mischbare organische Lösungsmittelsysteme; niedere Alkohole, wie z.B. Methanol, Ethanol, Isopropanol; niedere Ketone, wie z.B. Aceton; Glycerol, Polyethylenglykole, 1,2-Propylenglykol, Tetrahydrofurfurylalkoholpolyethylenglykolether (Tetraglykol); niedere Ether; niedere Ester; sowie deren Mischungen.

Je nach lipophilem bzw. hydrophilem Charakter des Arzneistoffs kann sich dabei vor der Vermischung mit dem, vorteilhaft in wäßriger Lösung vorliegenden, hydrophilen Peptid, eine klare Lösung oder eine Suspension des schwer wasserlöslichen Arzneistoffes ergeben.

Die mit diesem einfachen Verfahren erhaltenen, stabilen Lösungen des schwer wasserlöslichen Arzneistoffes können bereits als arzneiliche Zubereitungen angesehen und als solche auch verwendet werden.

Die Stabilität des wäßrig/organischen Systems ist unter in vitro wie auch in vivo Bedingungen jedoch nicht an die Anwesenheit des organischen Lösungsmittels wie z.B. Alkohol gebunden, wie man zunächst vermuten könnte. Diese Tatsache läßt sich durch Entfernen des Alkohols, z.B. durch Evaporation leicht zeigen, im Gegensatz zu handelsüblichen Arzneizubereitungen (flüssige Arzneizubereitungen zur oralen oder peroralen Einnahme, wie z.B. Tropfen) des Standes der Technik.

Dem organischen Zusatz in den erfindungsgemäßen Produkten dürfte damit eine Vehikelfunktion zukommen, indem beispielsweise durch Veränderung der Hydrathülle der von Kollagen abgeleiteten Substanz, wie Gelatine, deren lipophile Bezirke aktiviert werden. Auf diese Weise können bevorzugt Wechselwirkungen zwischen der Gelatine und lipophilen Molekülteilen eines schwer wasserlöslichen Arzneistoffmoleküls stattfinden.

Eine Modifizierung des unter a) beschriebenen Verfahrensschrittes ergibt sich im Falle von hydrophilen Peptiden, die sich in kaltem Wasser (Wasser von Raumtemperatur) auflösen, wie z.B. Kollagenhydrolysaten oder Gelatinederivaten. Diese Stoffe können beispielsweise direkt mit einer wäßrig/alkoholischen Lösung eines schwer wasserlöslichen Arzneistoffs vermischt und somit darin aufgelöst werden.

Eine weitere Ausführungsform des unter a) beschriebenen Verfahrensschrittes zur Herstellung von flüssigen, wäßrigen und stabilen Lösungen eines Arzneistoffes im Sinne der Erfindung kann dann angewendet werden, wenn dieser im sauren oder basischen Medium beispielsweise unter Salzbildung löslich ist. Bei dieser Verfahrensvariante kann auf organische Lösungsmittel teilweise, aber überraschenderweise auch ganz verzichtet werden.

Die erfindungsgemäße Vorgehensweise soll hier beispielhaft an einem Arzneistoff dargestellt werden, der im basischen Medium unter Salzbildung in Lösung überführt werden kann:

Vorzugsweise wird ein solcher Arzneistoff im wäßrig-ammoniakalischen Medium bei pH-werten oberhalb von 7 gelöst und mit dem hydrophilen Peptid, oder wägrigen Lösungen davon, homogen vermischt, so daß eine klare Lösung entsteht. Ist der Arzneistoff zersetzlich, z.B durch Kohlendioxidzutritt, wird vorteilhaft unter Inertgasatmosphäre (z.B. Stickstoff- oder Argonbegasung) gearbeitet.

Die Salzbildung kann danach unter Säurezusatz rückgängig gemacht werden, oder das Ammoniak bevorzugt durch die im unten beschriebenen Verfahrensachritt der Trocknung (b) angegebenen Methoden, z.B. durch einfache Evaporation oder Gefriertrocknung entfernt werden.

Bei Arzneistoffen mit Basencharakter ist umgekehrt zu verfahren.

Nicht jeder Arzneistoff kann jedoch unter Salzbildung in wäßrigem Medium in Lösung gebracht werden, da auch Arzneistoffsalze schwer wasserlöslich sein können. Trotzdem lassen sich in einer weiteren Ausbildungsform des erfindungsgemäßen Verfahrensschrittes a) unter Salzbildung in den obengenannten organischen Lösungsmitteln, insbesonderee Polyethylenglykolen, 1,2-Propylenglykol oder Tetrahydrofurfurylalkoholpolyethylenglykolether (Tetraglykol) von solchen Stoffen Lösungen herstellen. So ist beispielsweise von Glibenclamid oder Indometacin bekannt, daß man diese Stoffe mittels Ammoniakzusatz in Polyethylenglykolen lösen kann.

Gibt man diese (wasserfreien) Lösungen von Arzneistoff-Salzen in eine wäßrige Lösung des hydrophilen Peptids, die zuvor auf einen pH-Wert eingestellt wurde, der die Salzbildung rückgängig macht, bzw. stellt man die wäßrige Lösung des hydrophilen Peptids erst nach der Zugabe der organischen Salzlösung auf einen pH-Wert ein, der die Salzbildung rückgängig macht erhält man ebenfalls erfindungsgemäße Arzneistoff-Lösungen.

Das verwendete organische Lösungsmittel braucht in diesem Fall nicht aus der Zubereitung entfernt zu werden, da ihm gleichzeitig eine Weichmacherfunktion zukommt, beispielsweise bei der erfindungsgemäßen Cryopellet-Herstellung mit Gelatine oder anderen von Kollagen abgeleiteten Substanzen.

Bei einer weiteren Ausbildungsform der Stufe a) des erfindungsgemäßen Verfahrens wird ein schwer wasserlöslicher Arzneistoff zunächst trocken mit dem hydrophilen Peptid vermischt und nach Zugabe eines der beschriebenen, flüchtigen, organischen Lösungsmittel (z.B. Alkohol) mit diesem geknetet. Nach dem Trocknen des Lösungsmittels erhält man eine pulverisierbare Masse mit den erfindungsgemäß beschriebenen Eigenschaften.

Im Verfahrensschritt a) können in allen beschriebenen Ausführungsformen des erfindungsgemäßen Verfahrens Dehydratationsmittel, wie z.B. die aufgeführten organischen Lösungsmittel verwendet werden. Solche Zusätze können nach Auflösung der tripelhelikalen Struktur, beispielsweise von Gelatine oder fraktionierter Gelatine eine trans-cis-Konformationsumstellung der nun freien alpha-Ketten bewirken, sodaß der Arzneistoff bevorzugt in Wechselwirkung mit den lipophilen Bezirken der Gelatine bzw. fraktionierten Gelatine treten kann (Konjugatbildung). Nach Rehydratisierung wird der betreffende Arzneistoff in der sehr rasch zurückgebildeten Ursprungskonformation der Gelatine inkorporiert.

Bei dem unter b) angegebenen Verfahrensschritt werden das oder die Lösungsmittel aus der in Stufe a) des erfindungsgemäßen Verfahrens erhaltenen Mischung entfernt. Dies kann beispielsweise durch Aufkonzentrierung, Evaporation, Trocknung oder Kombinationen der genannten Prozesse geschehen, wobei sich verschiedene Verfahrensvarianten ergeben können.

### Variante A:

In einer Ausbildungsform des erfindungsgemäßen Verfahrensschrittes (b) kann beispielsweise durch einfache Lufttrocknung und anschließende Mahlung ein Gelatinegranulat mit erfindungsgemäßen Eigenschaften erhalten werden. Bei der Trocknung kann zur Beschleunigung des Trocknungsvorgangs und zur Einhaltung von niedrigen Temperaturen unter Vakuum (ca. 3000 bis 5000 Pa (ca. 30 bis 50 mbar)), beispielsweise bei Trocknungstemperaturen von bis zu 50°C gearbeitet werden.

### Variante B:

Durch übliche Sprühtrocknung werden Wasser bzw. ein flüchtiges, organisches Lösungsmittel bzw. das Ammoniak entfernt. Man gewinnt ein üblicherweise trockene Pulver. In herkömmlichen Sprühtrocknungsanlagen verwendet man normalerweise als Prozeßgas Luft mit einer Eintrittstemperatur zwischen 100°C und 300°C, wobei sich eine mittlere Temperaturdifferenz zwischen Prozeßgas und Gut von 50°C bis 100°C ergeben kann. Als Prozeßgas kann bei Vorliegen von besonders oxidationsempfindlichen Substanzen auch ein Inertgas, wie z.B. Stickstoff eingesetzt werden.

Organische Lösungsmittel können vor der Sprühtrocknung entfernt werden, z.B. durch Evaporation und können so einfach und wirtschaftlich zur erneuten Verwendung wiedergewonnen werden.

### Variante C:

Die Zubereitung wird durch übliche Gefriertrocknung in den trockenen, festen Zustand überführt.

Das Eingefrieren der Zubereitung kann beispielsweise in der Gefriertrocknungsanlage selbst erfolgen, z.B. im Bereich von -10°C bis -40°C. Das vollständige Eingefrieren kann durch eine sprunghafte Änderung der Leitfähigkeit in der zu gefrierenden Probe leicht festgestellt werden. Die eigentliche Trocknung wird bei Temperaturen von 15°C unterhalb des Sublimationspunktes von Wasser bei einem Druck von 0,1 Pa bis 10² Pa (0,001 mbar bis 1,03 mbar) durchgeführt. Der Trocknungsvorgang, der in einer herkömmlichen Gefriertrocknungsanlage (Kondensatortemperatur: ca. -40°C) bei -25°C und 33 Pa (0,33 mbar) in der Primärtrocknung unter Sublimation des gefrorenen Wassers ablaufen kann, führt nach Sekundärtrocknung (Desorption) zu einem gefriergetrockneten Produkt.

Organische Lösungsmittel können vor der Sprühtrocknung entfernt werden, z.B. durch Evaporation und können so einfach und wirtschaftlich zur erneuten Verwendung wiedergewonnen werden.

Flüssige, erfindungsgemäße Zubereitungen von schwer wasserlöslichen Arzneistoffen lassen sich in einer weiteren Ausführungsform des erfindungsgemäßen Verfahrens zu festen oder halbfesten bzw. gelförmigen Formkörpern weiterverarbeiten. Solche Zubereitungen zeichnen sich durch eine stabile Fixierung des molekulardispersen Zustands des schwer wasserlöslichen Arzneistoffes in einer Matrix aus dem hydrophilen Peptid, insbesondere von Kollagen abgeleiteten Substanzen, aus. Sie lassen sich im wäßrigen bzw. physiologischen Milieu vollständig in einen stabilen, flüssigen, gelösten Zustand zurückführen (redispergieren).

Bevorzugt lassen sich derartige Zubereitungen direkt, oder nach Aufkonzentrieren, mittels des Cryopel^{R}-Verfahrens (Messer Griesheim GmbH) zu den in zahlreichen Patentanmeldungen (z. B. der Patentanmeldung P 42 01 179.5 vom 17.01.1992) der ALFATEC-Pharma GmbH beschriebenen Cryopellets in flüssigem Stickstoff formen und anschließend gefriertrocknen. Auf diese Weise gewinnt man, unter Erhalt der erfindungsgemäßen Eigenschaften, nach einem, für empfindliche Arzneistoffe geeigneten, schonenden Verfahren gefriergetrocknete Cryopellets, die sich beispielsweise aufgrund ihrer Lagerstabilität, hohen mechanischen Stabilität, guten Fließeigenschaften, variabel gestaltbaren Durchmessers von 0,2 - 12 mm u.a. entweder in konventionelle Hartgelatinekapseln abfüllen lassen oder in Dosierspendern abgefüllt als einzeldosierte Akutformen verwenden lassen.
Durch geeignete Hilfsstoffzusätze, wie z.B. Mannit können mittels Gefriertrocknung extrem schnell in kaltem Wasser auflösbare Zubereitungen ("feste Tropfen") hergestellt werden.

Weist das hydrophile Peptid sol-gel bildende Eigenschaften auf, wie beispielsweise bei Verwendung von Gelatine oder fraktionierter Gelatine mit einem Maximum der Molekulargewichtsverteilung oberhalb von ca. 9,5 x 10⁴ D lassen sich durch Zusatz der beschriebenen Weichmacher halbfeste bis gelförmige Konsistenzen der Zubereitung einstellen, wie sie auch von konventionellen Weichgelatinekapseln her bekannt sind.

Das Mengenverhältnis des hydrophilen Peptids zu Weichmacher kann von 1:0,001 bis 1:50, insbesondere von 1:0,1 bis 1:5, angegeben in Gewichtsteilen, variiert werden.

Dabei zeigt sich, daß die Weichmachersubstanzen, die chemisch gesehen zur Gruppe der Polyole gehören, neben einem Einfluß auf die Hydrathülle des hydrophilen Peptids wie z.B Gelatine oder fraktionierte Gelatine, verbunden mit einer Aktivierung lipophiler Molekülbezirke, auch die Stabilisierung und den Grad helikaler Konformation dieser Moleküle bestimmen können. Dadurch können noch intensivere Wechselwirkungen (Konjugatbildung) zwischen der Gelatine bzw. fraktionierten Gelatine und lipophilen Molekülteilen eines erfindungsgemäßen Arzneistoffes stattfinden. Erstaunlicherweise kann somit der Beladungsgrad der erfindungsgemäßen Matrix mit Arzneistoff sehr hoch gewählt werden (bis 0,5:1), ohne daß sich Probleme mit der Stabilität der molekulardispersen Verteilung ergeben.

Besonders schonend und zu einem optisch ansprechenden Produkt mit erstaunlicher technologischer Vielfalt und Vorteilen läßt sich eine solche erfindungsgemäße, Zubereitung zu Cryopellets formen, die durch direktes Eintropfen bzw. Eindosieren der zu verarbeitenden Masse in ein Tauchbad mit Flüssigstickstoff, z.B. mittels des CryopelR-Verfahrens (siehe oben) hergestellt werden. Im Bedarfsfall kann durch Trocknung solcher Cryopellets ein Produkt erhalten werden, das sich vorteilhaft in Hartgelatinekapseln abfüllen läßt, optisch sehr ansprechend ist, und auf einfachem und wirtschaftlichem Wege gewonnen wird, unter Erhalt der erfindungsgemäßen Eigenschaften.

Solche halbfesten bis gelförmigen Zubereitungen können auch als Füllgut, sowohl für konventionelle Weichgelatinekapseln dienen, als auch als Füllgut für Weichgelatinekapseln, die durch Eindosieren in Flüssigstickstoff geformt werden. Halbfeste Massen sind auch in andere geeignete Behältnisse, wie z.B. gekühlte Blister oder ähnliche Hohlformen eindosierbar. Die Blister können anschließend direkt versiegelt werden und man gewinnt einzeldosierbare Arzneiformen.

Neben den bisher bereits erwähnten Ausführungsformen des erfindungsgemäßen Verfahrens ergibt sich eine weitere Ausführungsform aus der Kenntnis über die Qualität des eingesetzten hydrophilen Peptids.

Bezüglich von Kollagen abgeleiteten Substanzen ist beispielsweise bekannt, daß ihr Aschegehalt, d.h. der Entsalzungsgrad in Abhängigkeit von der Herstellung variiert. Bei handelsüblichen Gelatinesorten kann der Aschegehalt bis zu 2 Gew.% betragen. Bei guten Qualitäten liegt der Restaschegehalt jedoch herstellungsbedingt bei Werten unterhalb von 0,2 Gew.%.

Andererseits ist von schwer wasserlöslichen Arzneistoffen, z.B. von Arzneistoffen mit polarem Charakter bekannt, daß sie mit Calcium- oder Magnesiumionen Komplexe bilden können. Diese Zusätze von zweiwertigen Ionen bewirken durch Wechselwirkung mit den polaren Gruppen dieser Stoffe eine Konjugat-(Komplexbildung) der beiden Komponenten.

Wechselwirkungen solcher Komplexe aus schwer wasserlöslichen Arzneistoffen mit Calcium- oder Magnesiumsalzen, mit den funktionellen Gruppen eines hydrophilen Peptids führen zu einer wirkungsvollen Stabilisierung des molekulardispersen Zustands im Sinne der Erfindung, die ohne das hydrophile Peptid nicht möglich wäre und daher zu Ausfällungen des schwer wasserlöslichen Arzneistoffs im physiologischen Milieu führen würde.

Daher wird bei dieser Ausführungsform des erfindungsgemäßen Verfahrens nun beispielsweise eine handelsübliche Gelatine mit einem höheren Aschegehalt (Salzgehalt) verwendet. Andererseits kann einer herstellungsbedingt vollentsalzten Gelatinesorte eine definierte Menge von Calcium- oder Magnesium-salzen (z.B. 1 bis 2 Gew.% Calciumchlorid oder Magnesium-chlorid) kontrolliert zugesetzt werden.

Außerdem fördern die Calciumionen durch Komplexbildung mit Bestandteilen der Zellmembran die Resorption des schwer wasserlösliche Arzneistoffes.

Die erfindungsgemäßen Zubereitungen eines schwer wasserlöslichen Arzneistoffs können sowohl für pharmazeutische, kosmetische als auch für diätetische (health care) Zwecke geeignet sein.

Unter einer pharmazeutischen Zubereitung im Sinne der Erfindung werden sowohl Arzneimittelzubereitungen, als auch kosmetische oder diätetische Lebensmittelzubereitungen (health care) verstanden.

Pulverförmige Zubereitungen können durch Konfektionierung in geeignete Behältnisse (z.B. Beutel) zur Herstellung von flüssigen, wäßrigen, einnehmbaren Arzneilösungen dienen. Das Pulver kann ebenso in übliche Hartgelatinekapseln abgefüllt werden, zu klassischen Pellets oder Granulaten weiterverarbeitet werden und dann in Hartgelatinekapseln abgefüllt werden. Komprimierung nach üblichen Methoden zu Tabletten, Herstellung von Dragees , Herstellung von magensaftresistent überzogenen Arzneiformen etc., ggf. unter Anwendung üblicher Tablettierhilfsstoffe, wie z.B. FST-Komplex, ist ebenfalls möglich. Vorteilhafterweise sind erfindungsgemäße Zubereitungen, die von Kollagen abgeleitete Substanzen wie z.B Kollagenhydrolysate enthalten, zur Direkttablettierung geeignet.

Erfindungsgemäß lyophilisierte Zubereitungen, besonders bei Verwendung niedermolekularer Gelatinesorten, Kollagenhydrolysaten oder Gelatinederivaten lassen sich in kaltem Wasser beschleunigt wiederauflösen. Solche Zubereitungen können auch als Basis für sterile Arzneiformen wie z.B. Parenteralia oder Ophthalmika dienen. Dabei können als Behältnisse für Injektionslösungen z.B. übliche Zweikammerspritzen verwendet werden.

Parenterale Arzneiformen im Sinne der Erfindung zeichnen sich aufgrund der einfachen Zusammensetzung des Systems, unter Verwendung von unbedenklichen, und für i.v. Zwecke bewährten Hilfsstoffen besonders durch hohe. Verträglichkeit aus.

Auch transdermale Zubereitungen bzw. Verarbeitung einer erfindungsgemäßen Zubereitung mit üblichen Hilfsstoffen zu einer transdermalen Zubereitung ist möglich.

Erfindungsgemäße Arzneizubereitungen lassen sich auch zur nasalen oder pulmonalen Applikation einsetzen. Pulverförmige Systeme können beispielsweise zur Herstellung von Inhalations-Arzneiformen dienen. Cryopellets können sich bevorzugt für nasale Applikationszwecke eignen, z.B. aufgrund ausgeprägter bioadhäsiver Eigenschaften.

Darüberhinausgehend können die erfindungsgemäßen Systeme in Abhängigkeit der eingesetzten, zu solubilisierende Substanzen auch als diätetische Lebensmittel (health care) Verwendung finden oder in technischen Bereichen zur Anwendung kommen.

Durch die vorliegende Erfindung ist es ebenfalls möglich, auch schwer wasserlösliche, synthetische bzw. pflanzliche Süßstoffe, z.B. zur Verwendung als diätetisches Lebensmittel, in eine lösungsvermittelte Form zu bringen. Von Steviosid, einem Diterpensäure-glucosyl-sophorosid aus Stevia rebaudiana, ist beispielsweise bekannt, daß die Süßwirkung gegenüber Saccharose zwar um das 150 bis 300-fache stärker ist, die Substanz sich selbst in heißen Getränken aber schlecht auflöst. In cryopelletierter Form vorliegendes Steviosid, das erfindungegemäß lösungsvermittelt ist, läßt sich besonders vorteilhaft in Dosierspender zur Einzelentnahme konfektionieren.

Im Rahmen seines Fachwissens kann der Fachmann leicht selbständig weitere Verfahrensvarianten ausarbeiten. Folgende Beispiele sollen die Erfindung näher erläutern:

### Beispiel 1:

1400 g Gelatinepulver (30 Bloom) werden in 14 l dest. Wasser 20 min vorgequollen und danach bei 40°C eine Lösung hergestellt.

100 g Hydrochlorothiazid werden in 7 l Ethanol gelöst und mit der Gelatinelösung homogen vermischt, bis eine klare Lösung entsteht.

Nach Evaporation des Ethanols wird durch Sprühtrocknung, bei der die Eintrittstemperatur des Prozessgases bis zu 180°C und die Austrittstemperatur 80°C beträgt, wird Lösungsmittel entzogen und man erhält ein Pulver.

150 mg des Pulvers (entsprechend 10 mg Hydrochlorothiazid) werden in 25 ml künstlichem Magensaft bei 37°C wiederaufgelöst. Es ergibt sich eine klare Lösung.

Das erhaltene Pulver wird nach Trockengranulation mit üblichen Tablettierhilfsstoffen (FST-Komplex) gemischt und auf einer Tablettenmaschine zu Tabletten mit einem Gehalt von 10 mg Hydrochlorothiazid verpreßt.

### Beispiel 2:

Die Durchführung erfolgt analog Beispiel 1 unter Verwendung eines Gelatinepulvers mit 150 Bloom.

Das analog Beispiel 1 erhaltene Pulver wird nach Vermischen mit Lactose als Füllstoft zu Hartgelatinekapseln mit einem Gehalt von 10 mg Hydrochlorothiazid weiterverarbeitet.

### Beispiel 3:

150 g handelsübliches Kollagenhydrolysat
150 g Mannit
700 g dest. Wasser

Das Mannit und das Kollagenhydrolysat werden in 350 g des Wassers gelöst. Durch Zugabe von Ammoniaklösung wird ein pH-Wert zwischen 8 und 10 eingestellt.

50 g Quercetin werden in 350 g Wasser homogen verteilt und unter Stickstoffbegasung mittels Ammoniaklösung ein pH-Wert zwischen 8 und 10 eingestellt.

Beide Lösungen werden vermischt und durch Eintropfen der Masse in ein Tauchbad mit Flüssigstickstoff mittels des Cryopel^{R}-Dosiersystems werden Pellets mit einem Durchmesser von 5 mm geformt.

Die gefrorenen Pellets werden in einer Gefriertrocknungsanlage mit einer Primärtrocknung bei -25°C und 5 Pa (0,05 mbar) und einer Sekundärtrocknung bei 22°C getrocknet.

Die getrockneten Pellets können als einzeldosierbare Arzneiform in einen Dosierspender abgefüllt werden. Sie sind in kaltem Wasser leicht löslich, wobei sich eine klare Lösung ergibt.

## Patentansprüche

1. Schwer wasserlösliche Wirkstoffe enthaltende Zubereitung, gekennzeichnet durch eine molekulardisperse bis kolloiddisperse Verteilung des schwer wasserlöslichen Wirkstoffes in einem unvernetzten hydrophilen Peptid mit einem Molekulargewicht über 100 D.

2. Zubereitung nach Anspruch 1, gekennzeichnet durch ein hydrophiles Peptid aus der Gruppe: Kollagen, eine von Kollagen abgeleitete Substanz, eine Mischung aus von Kollagen abgeleiteten Substanzen, Gelatine, fraktionierte Gelatine, ein Kollagenhydrolysat, ein Gelatinederivat, ein Elastinhydrolysat; ein Pflanzenprotein, ein Pflanzenproteinhydrolysat, Albumine, Casein, Caseinhydrolysate, sowie Mischungen daraus.

3. Zubereitung nach Anspruch 1 und/oder 2, dadurch gekennzeichnet, daß das Peptid ein Sol-Gel-Bilder ist.

4. Zubereitung nach Anspruch 2, dadurch gekennzeichnet, daß die Gelatine oder fraktionierte Gelatine ein Maximum der Molekulargewichtsverteilung oberhalb von 9,5 x 10⁴ D besitzt.

5. Zubereitung nach einem der Ansprüche 1 bis 4, dadurch gekennzeichnet, daß sie in flüssiger oder in halbfester bis gelförmiger Form vorliegt.

6. Zubereitung nach einem der Ansprüche 1 bis 4, dadurch gekennzeichnet, daß sie in fester Form, insbesondere in sprühgetrockneter oder in gefriergetrockneter Form vorliegt.

7. Zubereitung nach einem der Ansprüche 1 bis 6, enthaltend zusätzliche hydrophile Makromoleküle aus der Gruppe: Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylalkohol, Polyvinylpyrrolidon, Polyethylenglykole, Polyacrylsäure, und Polymere aus Methacrylsäure und Methacrylsäureestern; sowie deren Mischungen, in einem Gewichtsverhältnis zu dem hydrophilen Peptid bis zu 1:1.

8. Pharmazeutische Zubereitung von schwer wasserlöslichen Arzneistoffen, enthaltend eine Zubereitung von schwer wasserlöslichen Wirkstoffen nach einem der Ansprüche 1 bis 7 neben pharmazeutisch üblichen Träger- und Hilfsstoffen.

9. Zubereitung nach Anspruch 8, enthaltend als zusätzliche Träger- und Hilfsstoffe:
zusätzliche Gerüstbildner, Füllstoffe, Tenside, pH-Korrigentien bzw. Puffersubstanzen, Farbstoffe und/oder aromatisierende Zusätze bzw. Geschmackskorrigentien.

10. Zubereitung nach Anspruch 9, dadurch gekennzeichnet, daß als zusätzliche Gerüstbildner Dextrane, Saccharose, Glycin, Lactose, Sorbitol, Polyvinylpyrrolidon, Mannit; als Füllstoffe Stärke; als Tenside Polysorbate; als pH-Korrigentien bzw. Puffersubstanzen Dinatriumcitrat, Dinatriumphosphat; als Farbstoffe Curcumin; als aromatisierende Zusätze bzw. Geschmackskorrigentien Fruchtauszüge, Fruchtsaftkonzentrate enthalten sind.

11. Zubereitung nach einem der Ansprüche 1 bis 10, dadurch gekennzeichnet, daß sie einen oder mehrere Weichmacher aus der Gruppe: Glycerol, Propylenglykol, Polyethylenglykole, Triacetin, Sorbitol, Sorbitangemische, Sorbitollösungen, Glucosesirup, Polyole, Zuckeralkohole; sowie deren Mischungen, enthält.

12. Zubereitung nach einem der Ansprüche 6 bis 11, dadurch gekennzeichnet, daß sie eine Weichgelatinekapsel ist.

13. Zubereitung nach einem der Ansprüche 1 bis 12, dadurch gekennzeichnet, daß dem hydrophilen Peptid ein die Helikalität des Peptids beeintlussender Zusatz, nämlich Glycerin, Polyethylenglykol und/oder Tenside zugesetzt werden.

14. Zubereitung nach einem der Ansprüche 1 bis 13, dadurch gekennzeichnet, daß sie übliche Hilfsstoffe zur Retardierung aus der Gruppe: Alginate, Cellulosederivate, Polyvinylpyrrolidon, natürliche oder modifizierte Stärken, Polyacrylsäure und Polymere aus Methacrylsäure und Methacrylsäureestern, hydrophile Gummen; sowie deren Mischungen enthält.

15. Zubereitung nach einem der Ansprüche 11 bis 14, dadurch gekennzeichnet, daß sie eine Kombination von wenigstens einem hydrophilen Peptid mit wenigstens einem Weichmacher enthält.

16. Zubereitung nach einem der Ansprüche 8 bis 15, dadurch gekennzeichnet, daß sie einen Peptidarzneistoff mit einem üblichen Penetrationsbeschleuniger (Enhancer) aus der Gruppe: Chelatoren, Surfactants, Non-Surfactants, Gallensaure Salze und ihre Derivate, Fettsäuren und ihre Derivate; enthält.

17. Zubereitung nach Anspruch 16, dadurch gekennzeichnet, daß sie als Chelator Ethylendiamintetraessigsäure (EDTA),Citronensäure, Salicylate, N-Acylderivate von Kollagen oder von Kollagen abgeleitete Substanzen, N-Aminoacylderivate von beta-Diketonen (Enamine), als Surfactant Natriumlaurylsulfat, Polyoxyethylen-9-laurylether, Polyoxyethylen-20-cetylether, als Non-Surfactant ungesättigtzyklische Harnstoffverbindungen, 1-Alkyl- und 1-Alkenyl-azacycloalkanon-Derivate, als gallensaures Salz bzw. ihr Derivat Natrium-deoxycholat, Natrium-glykocholat, Natriumtaurodihydrofusidat (STDHF), Natrium-glykodihydrofusidat, und/oder als Fettsäuren und ihr Derivat Ölsäure, Caprylsäure, Caprisäure, Acylcarnitine, Acylcholine und ihre Mono- und Diglyceride enthält.

18. Zubereitung nach einem der Ansprüche 8 bis 17, dadurch gekennzeichnet, daß sie eine Kombination aus dem hydrophilen Peptid und einat Lösungsvermittler aus der Gruppe: Polyethylenglykole, Tetrahydrofurfurylalkoholpolyethylenglykolether (Tetraglykol) und 1,2,-Propylenglykol enthält.

19. Zubereitung nach einem der Ansprüche 8 bis 18, dadurch gekennzeichnet, daß sie einen hydrotropen Stoff als Zusatz enthält.

20. Zubereitung nach Anspruch 19, dadurch gekennzeichnet, daß sie Harnstoff, N-Methylacetamid oder Nikotinsäure als hydrotropen Stoff enthält.

21. Verfahren zur Herstellung einer schwer wasserlösliche Wirkstoffe in disperser Verteilung enthaltenden Zubereitung nach einem der Ansprüche 1 bis 20, dadurch gekennzeichnet, daß man:
a) den schwer wasserlöslichen Wirkstoff in einem Lösungsmittel kolloidal oder molekular dispergiert und diese Dispersion mit einer wäßrigen Lösung eines hydrophilen Peptids mit einem Molekulargewicht über 100 D vermischt, wobei man das hydrophile Peptid nicht vernetzt, und
b) das oder die Lösungsmittel entfernt und so den Wirkstoff dispers verteilt.

22. Verfahren nach Anspruch 21, dadurch gekennzeichnet, daß man entweder in Stufe
a) den schwer wasserlöslichen Wirkstoff in einem mit Wasser mischbaren organischen Lösungsmittel, ausgewählt aus der Gruppe: mit Wasser mischbare organische Lösungsmittelsysteme; niedere Alkohole wie Methanol, Ethanol, Isopropanol; niedere Ketone wie Aceton; Glycerol, Polyethylenglykole, 1,2-Propylenglykol; Tetrahydrofurfurylalkoholpolyethylenglykolether (Tetraglykol), niedere Ether, niedere Ester, sowie deren Mischungen löst oder in Stufe a) den schwer löslichen Wirkstoff unter Salzbildung löst.

23. Verfahren nach Anspruch 22, dadurch gekennzeichnet, daß man im Anschluß an Stufe a) die Salzbildung wieder rückgängig macht.

24. Verfahren nach den Ansprüchen 21 bis 23, dadurch gekennzeichnet, daß man in Stufe b) alle Lösungsmittel, z. B. durch Sprühtrocknung oder Gefriertrocknung, entfernt.

25. Verfahren nach Anspruch 24, dadurch gekennzeichnet, daß man vor der Gefriertrocknung die Mischung aus schwer wasserlöslichem Wirkstoff und hydrophilem Peptid zu Cryopellets formt.

26. Verfahren zur Herstellung einer pharmazeutischen Zubereitung von schwer wasserlöslichen Arzneistoffen, dadurch gekennzeichnet, daß man im Verfahren nach einem der Ansprüche 21 bis 25 als schwer wasserlöslichen Wirkstoff einen schwer wasserlöslichen Arzneistoff einsetzt und in Stufe a) zusätzliche hydrophile Makromoleküle aus der Gruppe: Agar-Agar, Gummi arabicum, Pektine, Traganth, Xanthan, natürliche sowie modifizerte Stärken, Dextrane, Dextrine, Maltodextrin, Chitosan, Alginate, Cellulosederivate, Polyvinylpalkohol, Polyvinylpyrrolidon, Polyethylenglykole, Polyacrylsäure, und Polymere aus Mehacrylsäure und Mathacrylsäureestern; sowie deren Mischungen, in einem Gewichtsverhältnis zu dem hydrophilen Peptid bis zu 1:1 zusetzt und in Stufe a) oder nach Stufe b) pharmazeutisch übliche Träger- und Hilfsstoffe zusetzt.

27. Verfahren nach Anspruch 26, dadurch gekennzeichnet, daß man der Mischung, die wenigstens einen schwer wasserlöslichen Arzneistoff und ein hydrophiles Peptid enthält, einen oder mehrere Weichmacher aus der Gruppe: Glycerol, Propylenglykol, Polyethylenglykole, Triacetin, Sorbitol, Sorbitangemische, Sorbitollösungen, Glucosesirup, Polyole, Zuckeralkohole; sowie deren Mischungen, zusetzt.

28. Verfahren nach Anspruch 26 und/oder 27, dadurch gekennzeichnet, daß man aus der Mischung, die wenigstens einen schwer wasserlöslichen Arzneistoff, ein hydrophiles Peptid und einen Weichmacher enthält, durch Eintropfen in Flüssigstickstoff Weichgelatinekapseln formt.

29. Verfahren nach Anspruch 26 und/oder 27, dadurch gekennzeichnet, daß man aus der Mischung, die wenigstens einen schwer wasserlöslichen Arzneistoff, ein hydrophiles Peptid und einen Weichmacher enthält, durch Eintropfen in Flüssigstickstoff Cryopellets formt.

## Claims

1. Preparation containing slightly water-soluble active ingredients, characterized by the slightly water-soluble active ingredient being molecularly or colloidally dispersed in an uncrosslinked hydrophilic peptide with a molecular weight above 100 D.

2. Preparation according to Claim 1, characterized by a hydrophilic peptide from the group: collagen, a substance derived from collagen, a mixture of substances derived from collagen, gelatin, fractionated gelatin, a collagen hydrolysate, a gelatin derivative, an elastin hydrolysate; a vegetable protein, a vegetable protein hydrolysate, albumins, casein, casein hydrolysates, and mixtures thereof.

3. Preparation according to Claim 1 and/or 2, characterized in that the peptide is a sol-gel former.

4. Preparation according to Claim 2, characterized in that the gelatin or fractionated gelatin has a maximum in the molecular weight distribution above 9.5 × 10⁴ D.

5. Preparation according to any of Claims 1 to 4, characterized in that it is in liquid form or in semisolid to gel form.

6. Preparation according to any of Claims 1 to 4, characterized in that it is in solid form, in particular in spray-dried or in freeze-dried form.

7. Preparation according to any of Claims 1 to 6, containing additional hydrophilic macromolecules from the group: agar-agar, gum arabic, pectins, tragacanth, xanthan, natural and modified starches, dextrans, dextrins, maltodextrin, chitosan, alginates, cellulose derivatives, polyvinyl alcohol, polyvinylpyrrolidone, polyethylene glycols, polyacrylic acid, and polymers of methacrylic acid and methacrylic esters; and mixtures thereof, in a ratio by weight with respect to the hydrophilic peptide of up to 1:1.

8. Pharmaceutical preparation of slightly water-soluble medicinal substances, containing a preparation of slightly water-soluble active ingredients according to any of Claims 1 to 7 in addition to pharmaceutically customary carriers and ancillary substances.

9. Preparation according to Claim 8, containing as additional carriers and ancillary substances:
additional bulking agents, fillers, surfactants, pH correctives or buffer substances, colours and/or flavouring additions or masking flavours.

10. Preparation according to Claim 9, characterized in that dextrans, sucrose, glycine, lactose, sorbitol, polyvinylpyrrolidone, mannitol are present as additional bulking agents; starch as fillers; polysorbates as surfactants; disodium citrate, disodium phosphate as pH correctives or buffer substances; curcumin as colours; fruit extracts, fruit juice concentrates as flavouring additions or masking flavours.

11. Preparation according to any of Claims 1 to 10, characterized in that it contains one or more plasticizers from the group: glycerol, propylene glycol, polyethylene glycols, triacetin, sorbitol, sorbitan mixtures, sorbitol solutions, glucose syrup, polyols, sugar alcohols; and mixtures thereof.

12. Preparation according to any of Claims 6 to 11, characterized in that it is a soft gelatin capsule.

13. Preparation according to any of Claims 1 to 12, characterized in that an addition influencing the helicality of the peptide, namely glycerol, polyethylene glycol and/or surfactants, are added to the hydrophilic peptide.

14. Preparation according to any of Claims 1 to 13, characterized in that it contains conventional ancillary substances for slowing release from the group: alginates, cellulose derivatives, polyvinylpyrrolidone, natural or modified starches, polyacrylic acid and polymers of methacrylic acid and methacrylic esters, hydrophilic gums; and mixtures thereof.

15. Preparation according to any of Claims 11 to 14, characterized in that it contains a combination of at least one hydrophilic peptide with at least one plasticizer.

16. Preparation according to any of Claims 8 to 15, characterized in that it contains a peptide medicinal substance with a conventional penetration accelerant (enhancer) from the group: chelators, surfactants, nonsurfactants, bile acid salts and derivatives thereof, fatty acids and derivatives thereof.

17. Preparation according to Claim 16, characterized in that it contains as chelator ethylenediaminetetraacetic acid (EDTA), citric acid, salicylates, N-acyl derivatives of collagen or substances derived from collagen, N-aminoacyl derivatives of beta-diketones (enamines), as surfactant sodium lauryl sulphate, polyoxyethylene 9 lauryl ether, polyoxyethylene 20 cetyl ether, as nonsurfactant unsaturated cyclic urea compounds, 1-alkyl- and 1-alkenyl-azacycloalkanone derivatives, as bile acid salt or derivative thereof sodium deoxycholate, sodium glycocholate, sodium taurodihydrofusidate (STDHF), sodium glycodihydrofusidate, and/or as fatty acids and derivative thereof oleic acid, caprylic acid, capric acid, acylcarnitines, acylcholines and mono- and diglycerides thereof.

18. Preparation according to any of Claims 8 to 17, characterized in that it contains a combination of the hydrophilic peptide and a solubilizer from the group: polyethylene glycols, tetrahydrofurfuryl alcohol polyethylene glycol ether (tetraglycol) and 1,2,-propylene glycol.

19. Preparation according to any of Claims 8 to 18, characterized in that it contains a hydrotropic substance as addition.

20. Preparation according to Claim 19, characterized in that it contains urea, N-methylacetamide or nicotinic acid as hydrotropic substance.

21. Process for producing a preparation containing slightly water-soluble active ingredients in dispersions, according to any of Claims 1 to 20, characterized in that:
a) the slightly water-soluble active ingredient is colloidally or molecularly dispersed in a solvent, and this dispersion is mixed with an aqueous solution of a hydrophilic peptide with a molecular weight above 100 D, the hydrophilic peptide not being crosslinked, and
b) the solvent(s) is (are) removed and thus the active ingredient is dispersed.

22. Process according to Claim 21, characterized in that either in stage
a) the slightly water-soluble active ingredient is dissolved in a water-miscible organic solvent selected from the group: water-miscible organic solvent systems; lower alcohols such as methanol, ethanol, isopropanol; lower ketones such as acetone; glycerol, polyethylene glycols, 1,2-propylene glycol; tetrahydrofurfuryl alcohol polyethylene glycol ether (tetraglycol), lower ethers, lower esters, and mixtures thereof, or in stage a) the slightly soluble active ingredient is dissolved with salt formation.

23. Process according to Claim 22, characterized in that the salt formation is reversed following stage a).

24. Process according to Claims 21 to 23, characterized in that in stage b) all solvents are removed, for example by spray drying or freeze drying.

25. Process according to Claim 24, characterized in that before the freeze drying the mixture of slightly water-soluble active ingredient and hydrophilic peptide is shaped to cryopellets.

26. Process for producing a pharmaceutical preparation of slightly water-soluble medicinal substances, characterized in that in the process according to any of Claims 21 to 25 a slightly water-soluble medicinal substance is employed as slightly water-soluble active ingredient, and in stage a) additional hydrophilic macromolecules from the group: agar-agar, gum arabic, pectins, tragacanth, xanthan, natural and modified starches, dextrans, dextrins, maltodextrin, chitosan, alginates, cellulose derivatives, polyvinyl alcohol, polyvinylpyrrolidone, polyethylene glycols, polyacrylic acid, and polymers of methacrylic acid and methacrylic esters; and mixtures thereof, are added in a ratio by weight with respect to the hydrophilic peptide of up to 1:1, and in stage a) or after stage b) pharmaceutically customary carriers and ancillary substances are added.

27. Process according to Claim 26, characterized in that one or more plasticizers from the group: glycerol, propylene glycol, polyethylene glycols, triacetin, sorbitol, sorbitan mixtures, sorbitol solutions, glucose syrup, polyols, sugar alcohols; and mixtures thereof, are added to the mixture containing at least one slightly water-soluble medicinal substance and a hydrophilic peptide.

28. Process according to Claim 26 and/or 27, characterized in that soft gelatin capsules are formed from the mixture containing at least one slightly water-soluble medicinal substance, a hydrophilic peptide and a plasticizer by dropwise introduction into liquid nitrogen.

29. Process according to Claim 26 and/or 27, characterized in that cryopellets are formed from the mixture containing at least one slightly water-soluble medicinal substance, a hydrophilic peptide and a plasticizer by dropwise introduction into liquid nitrogen.

## Revendications

1. Préparation contenant des substances actives difficilement solubles dans l'eau, caractérisée par une distribution en dispersion moléculaire à dispersion colloïdale de la substance active difficilement hydrosoluble dans un peptide hydrophile non réticulé de poids moléculaire supérieur à 100 D.

2. Préparation suivant la revendication 1, caractérisée par un peptide hydrophile du groupe : collagène, une substance dérivée du collagène, un mélange de substances dérivées du collagène, la gélatine, une gélatine fractionnée, un hydrolysat de collagène, un dérivé de gélatine, un hydrolysat d'élastine ; une protéine végétale, un hydrolysat de protéine végétale, des albumines, la caséine, des hydrolysats de caséine, ainsi que des mélanges de ces peptides.

3. Préparation suivant la revendication 1 et/ou la revendication 2, caractérisée en ce que le peptide est une substance pouvant former un sol-gel.

4. Préparation suivant la revendication 2, caractérisée en ce que la gélatine ou la gélatine fractionnée a un maximum de distribution de poids moléculaire au-dessus de 9,5 x 10⁴ D.

5. Préparation suivant l'une des revendications 1 à 4, caractérisée en ce qu'elle se présente sous la forme liquide ou allant de la forme semi-solide à la forme gélifiée.

6. Préparation suivant l'une quelconque des revendications 1 à 4, caractérisée en ce qu'elle se présente sous la forme solide, notamment sous une forme séchée par atomisation ou lyophilisée.

7. Préparation suivant l'une des revendications 1 à 6, contenant des macromolécules hydrophiles additionnelles du groupe : agar-agar, gomme arabique, pectines, gomme adragante, xanthane, amidons naturels ainsi que modifiés, dextranes, dextrines, maltodextrine, chitosane, alginates, dérivés de cellulose, poly(alcool vinylique), polyvinylpyrrolidone, polyéthylèneglycols, poly(acide acrylique) et polymères d'acide méthacrylique et d'esters d'acide méthacrylique ; ainsi que leurs mélanges, dans un rapport en poids avec le peptide hydrophile allant jusqu'à 1:1.

8. Préparation pharmaceutique de substances médicamenteuses difficilement solubles dans l'eau, contenant une préparation de substances actives difficilement solubles dans l'eau suivant l'une des revendications 1 à 7 à côté de supports et substances auxiliaires d'emploi classique en pharmacie.

9. Préparation suivant la revendication 8, contenant comme supports et substances auxiliaires additionnels : des agents additionnels structurants, des charges, des agents tensioactifs, des correcteurs de pH ou des substances tampon, des colorants et/ou des additifs aromatisants et des correcteurs de goût.

10. Préparation suivant la revendication 9, caractérisée en ce qu'elle contient comme agents additionnels structurants des dextranes, du saccharose, de la glycine, du lactose, du sorbitol, de la polyvinylpyrrolidone, du mannitol ; comme charges, de l'amidon ; comme tensioactifs, des polysorbates ; comme correcteurs de pH ou substances tampon, du citrate disodique, du phosphate disodique ; comme colorants, du curcumin ; comme additifs aromatisants ou correcteurs de goût, des extraits de fruits, des concentrés de jus de fruits.

11. Préparation suivant l'une des revendications 1 à 10, caractérisée en ce qu'elle contient un ou plusieurs émollients du groupe : glycérol, propylèneglycol, polyéthylèneglycols, triacétine, sorbitol, mélanges de sorbitanne, solutions de sorbitol, sirop de glucose, polyols, sucre-alcools ; ainsi que leurs mélanges.

12. Préparation suivant l'une des revendications 6 à 11, caractérisée en ce qu'elle est une capsule de gélatine molle.

13. Préparation suivant l'une des revendications 1 à 12, caractérisée en ce qu'un additif influant sur la forme en hélice du peptide, à savoir de la glycérine, un polyéthylèneglycol et/ou des agents tensioactifs, sont ajoutés au peptide hydrophile.

14. Préparation suivant l'une quelconque des revendications 1 à 13, caractérisée en ce qu'elle contient des substances auxiliaires classiques de retardement du groupe : alginates, dérivés cellulosiques, polyvinylpyrrolidone, amidons naturels et modifiés, poly(acide acrylique) et polymères d'acide méthacrylique et d'esters d'acide méthacrylique, gommes hydrophiles ; ainsi que des mélanges de ces substances.

15. Préparation suivant l'une des revendications 11 à 14, caractérisée en ce qu'elle contient une association d'au moins un peptide hydrophile avec au moins un émollient.

16. Préparation suivant l'une des revendications 8 à 15, caractérisée en ce qu'elle contient une substance médicamenteuse peptidique avec un accélérateur de pénétration (stimulateur) classique du groupe : chélateurs, surfactifs, non-surfactifs, sels d'acide gallique et leurs dérivés, acides gras et leurs dérivés.

17. Préparation suivant la revendication 16, caractérisée en ce qu'elle contient comme chélateur de l'acide éthylènediaminetétracétique (EDTA), de l'acide citrique, des salicylates, des dérivés N-acylés de collagène ou de substances dérivées de collagène, des dérivés N-aminoacylés de bêta-dicétones (énamines), comme surfactif du laurylsulfate de sodium, de l'éther laurylique de polyoxyéthylène-9, de l'éther cétylique de polyoxyéthylène-20, comme non-surfactif des composés d'urée cycliques non saturés, des dérivés de 1-alkyl- et 1-alcénylazacycloalcanones, comme sel d'acide gallique ou ses dérivés, du désoxycholate de sodium, du glycocholate de sodium, du taurodihydrofusidate de sodium (STDHF), du glycodihydrofusidate de sodium et/ou comme acides gras et leurs dérivés, de l'acide oléique, de l'acide caprylique, de l'acide caprique, des acylcarnitines, des acylcholines et leurs mono- et diglycérides.

18. Préparation suivant l'une des revendications 8 à 17, caractérisée en ce qu'elle contient une association formée du peptide hydrophile et d'un dissolvant du groupe des polyéthylèneglycols, d'un éther de polyéthylèneglycol d'alcool tétrahydrofurfurylique (tétraglycol) et du 1,2-propylèneglycol.

19. Préparation suivant l'une des revendications 8 à 18, caractérisée en ce qu'elle contient comme additif une substance hydrotrope.

20. Préparation suivant la revendication 19, caractérisée en ce qu'elle contient de l'urée, du N-méthylacétamide ou de l'acide nicotinique comme substance hydrotrope.

21. Procédé de production d'une préparation contenant des substances actives difficilement solubles dans l'eau distribuées en dispersion suivant l'une des revendications 1 à 20, caractérisé en ce que :
a) on met la substance active difficilement soluble dans l'eau en dispersion moléculaire ou colloïdale dans un solvant et on mélange cette dispersion avec une solution aqueuse d'un peptide hydrophile de poids moléculaire dépassant 100 D, sans réticuler le peptide hydrophile, et
b) on chasse le ou les solvants et on distribue ainsi la substance active en dispersion.

22. Procédé suivant la revendication 21, caractérisé en ce que dans l'étape a), on dissout la substance active difficilement soluble dans l'eau dans un solvant organique miscible à l'eau, choisi dans le groupe comprenant des systèmes de solvants organiques miscibles à l'eau ; des alcools inférieurs tels que le méthanol, l'éthanol, l'isopropanol ; des cétones inférieures telles que l'acétone ; le glycérol, des polyéthylèneglycols, le 1,2-propylèneglycol ; un éther de polyéthylèneglycol d'alcool tétrahydrofurfurylique (tétraglycol), des éthers inférieurs, des esters inférieurs ainsi que leurs mélanges ou bien, dans l'étape a), on dissout la substance active difficilement soluble par formation d'un sel.

23. Procédé suivant la revendication 22, caractérisé en ce qu'on fait rétrograder la formation de sel à la suite de l'étape a).

24. Procédé suivant les revendications 21 à 23, caractérisé en ce que dans l'étape b), on élimine tous les solvants, par exemple par séchage par atomisation ou bien par lyophilisation.

25. Procédé suivant la revendication 24, caractérisé en ce que le mélange de substance active difficilement soluble dans l'eau et de peptide hydrophile est transformé en cryopellets avant la lyophilisation.

26. Procédé de production d'une préparation pharmaceutique de substances médicamenteuses difficilement solubles dans l'eau, caractérisé en ce que dans le procédé suivant l'une des revendications 21 à 25, on utilise comme substance active difficilement soluble dans l'eau une substance médicamenteuse difficilement soluble dans l'eau et on ajoute dans l'étape a) des macromolécules hydrophiles additionnelles du groupe : agar-agar, gomme arabique, pectines, gomme adragante, xanthane, amidons naturels de même que modifiés, dextranes, dextrines, maltodextrine, chitosane, alginates, dérivés de cellulose, poly(alcool vinylique), polyvinylpyrrolidone, polyéthylèneglycols, poly(acide acrylique) et polymères d'acide méthacrylique et d'esters d'acide méthacrylique ; ainsi que leurs mélanges, dans un rapport en poids avec le peptide hydrophile allant jusqu'à 1:1, et on ajoute dans l'étape a) ou après l'étape b) des supports et substances auxiliaires d'emploi courant en pharmacie.

27. Procédé suivant la revendication 26, caractérisé en ce qu'on ajoute au mélange, qui contient au moins une substance médicamenteuse difficilement soluble dans l'eau et un peptide hydrophile, un ou plusieurs émollients du groupe : glycérol, propylèneglycol, polyéthylèneglycols, triacétine, sorbitol, mélanges de sorbitanne, solutions de sorbitol, sirop de glucose, polyols, sucres-alcools ; ainsi que des mélanges de ces émollients.

28. Procédé suivant la revendication 26 et/ou la revendication 27, caractérisé en ce qu'on forme des capsules de gélatine molle à partir du mélange qui contient au moins une substance médicamenteuse difficilement soluble dans l'eau, un peptide hydrophile et un émollient, en le faisant tomber en gouttes dans de l'azote liquide.

29. Procédé suivant la revendication 26 et/ou la revendication 27, caractérisé en ce qu'on forme des cryopellets à partir du mélange qui contient au moins une substance médicamenteuse difficilement soluble dans l'eau, un peptide hydrophile et un émollient, en le faisant tomber en gouttes dans de l'azote liquide.
